Europäisches Patentamt

European Patent Office

Office européen des brevets

(11) Publication number: **0 359 349**

**A2**

(12) **EUROPEAN PATENT APPLICATION**

(21) Application number: 89300121.4

(51) Int. Cl.5: **C09D 7/12 , D21H 19/58**

(22) Date of filing: 06.01.89

(30) Priority: 12.09.88 JP 227967/88

(43) Date of publication of application:
21.03.90 Bulletin 90/12

(84) Designated Contracting States:
DE FR GB

(71) Applicant: **JAPAN AS REPRESENTED BY DIRECTOR GENERAL AGENCY OF INDUSTRIAL SCIENCE AND TECHNOLOGY**
3-1, Kasumigaseki 1-chome Chiyoda-ku Tokyo100(JP)

Applicant: **SAN NOPCO LIMITED**
11 Ikkyonomoto-cho Higashiyama-ku Kyoto-shi Kyoto-fu(JP)

(72) Inventor: **Ito, Shoji**
450-5, Shimohirooka
Tsukuba-shi Ibaraki-ken(JP)
Inventor: **Mizoguchi, Kensaku**
98-181, Oaza Shimizu Fujishiro-machi
Kitasoma-gun Ibaraki-ken(JP)

Inventor: **Yamauchi, Aizo**
708 Namiki 3-chome
Tsukuba-shi Ibaraki-ken(JP)
Inventor: **Hayashi, Yoshihiro**
11-3, Nishinodai 3-chome
Chita-shi Aichi-ken(JP)
Inventor: **Watanabe, Tomoyuki**
17-8, Yahatashinmachi 1-chome
Chita-shi Aichi-ken(JP)
Inventor: **Daigo, Hiroshi**
127, Asakura-cho
Chita-shi Aichi-ken(JP)
Inventor: **Ikawa, Hideaki**
36-1, Akiba Nawa-cho
Tokai-shi Aichi-ken(JP)
Inventor: **Nishimura, Hideaki**
36-1, Akiba Nawa-cho
Tokai-shi Aichi-ken(JP)

(74) Representative: **Allard, Susan Joyce et al**
**BOULT, WADE & TENNANT 27 Furnival Street**
**London EC4A 1PQ(GB)**

(54) Thermoreversible thickener.

(57) The invention provides a thermoreversible thickener for aqueous dispersions such as latices, emulsions, water-borne coating materials for general uses, coating materials for coated paper and water-borne adhesives used in such a characteristic way that the addition thereof to an aqueous dispersion is carried out at a temperature below the transition temperature without increasing the viscosity of the aqueous dispersion and subsequent heating causes rapid irmobilization or thickening of the dispersion with thermoreversibility. The thickener of the invention comprises a homopolymer or a copolymer having reversibility of hydrophilicity and hydrophobicity obtained by the polymerization of an acrylamide-based monomer or monomers suitable to impart such a property.

## THERMOREVERSIBLE THICKENER

The present invention relates to a thickener or a thickening agent. More particularly, the invention relates to a thickener of the thermoreversible type useful for aqueous dispersions such as latices, emulsions, water-borne coating materials, water-borne adhesives and coating compositions for coated papers.

Traditionally, polymer-based thickeners or alkyl-modified polyether-based thickeners are used for aqueous dispersions. The thickeners of the former type include hydroxyethyl cellulose, carboxymethyl cellulose, sodium alginate, guar gum and sodium polyacrylate. Thickeners of the latter type are in general called associative thickeners. Each of these conventional thickeners has an inherent disadvantage in that the viscosity of the compositions compounded therewith is decreased as the temperature thereof is increased.

On the other hand, thickeners of another type generally called thermosensitive gelatinizing agents are known from the disclosures in Japanese Patent Kokai 57-10665, Japanese Patent Kokai 58-98327 and elsewhere. Thickeners of this type include poly(alkylene oxide), poly(vinyl methyl ether) and poly(alkylene oxide)-modified polysiloxanes. However, these thickeners have defects such as the low sensitivity to thickening response, difficulty in the optional setting of the temperature to begin thickening, difficulty in controlling the viscosity as the object and so on.

In view of the disadvantages inherent in the conventional thickeners described above, we have now developed a thickener which is able to control the viscosity of an aqueous dispersion throughout a wide range from a very low viscosity up to a non-flowable condition over a wide temperature range by the addition thereof to the aqueous dispersion. The thickener of the invention enables easy setting of the temperature at the beginning of thickening and still does not decrease the viscosity of an aqueous dispersion thickened by the use thereof in the course of temperature elevation.

Accordingly, the present invention provides a thermoreversible thickener for an aqueous dispersion comprising a polymer having thermoreversiblity of hydrophilicity and hydrophobicity.

The polymer of the thermoreversible thickener of the invention is a homopolymer or copolymer preferably selected from those belonging to the three classes described below:

1) a homopolymer or copolymer having thermoreversiblity of hydrophilicity and hydrophobicity obtained by the polymerization of an acrylamide monomer which gives a homopolymer capable of exhibiting thermoreversibility of hydrophilicity and hydrophobicity in an aqueous solution;

2) a copolymer having thermoreversibility of hydrophilicity and hydrophobicity obtained by the copolymerizaation of an acrylamide monomer capable of giving a homopolymer which exhibits thermoreversibility of hydrophilicity and hydrophobicity in an aqueous solution with another vinyl compound; and

3) a copolymer having thermoreversibility of hydrophilicity and hydrophobicity obtained by the copolymerization of a vinyl compound capable of giving a homopolymer which exhibits no thermoreversibility of hydrophilicity and hydrophobicity in an aqueous solution with a vinyl compound which gives a water-insoluble homopolymer, preferably, selected from vinyl compounds of the acrylamide-type.

By the term "a polymer having thermoreversibility of hydrophilicity and hydrophobicity" as used in this invention is meant such a polymer that, when a 1% by weight aqueous solution thereof is heated from a low temperature of, for example, $0\,^\circ C$ at a rate of temperature elevation of $1\,^\circ C$ per minute, the light transmittance of the solution at a wavelength of 500 nm is decreased by at least 50% in the course of temperature elevation up to boiling.

Preferably, these homopolymers and copolymers should have a intinsic viscosity of at least 0.3 dl/g.

The thickener of the invention is preferably used in such a way that the addition thereof to an aqueous dispersion is performed at a temperature not higher than the transition temperature thereof to thicken the aqueous dispersion by subsequently increasing the temperature of the dispersion.

The thermoreversible thickeners of the invention are particularly satisfactorily additives to coating compositions for coated paper or water-borne coating compositions for general uses.

The present invention relates to a thermoreversible thickener for an aqueous dispersion comprising a polymer having thermoreversibility between hydrophilicity and hydrophobicity.

In one of the preferred embodiments of the invention, the polymer is a homopolymer obtained by the polymerization of an acrylamide monomer which gives a homopolymer capable of exhibiting thermoreversibility of hydrophilicity and hydrophobicity in an aqueous solution. Typical examples of such acrylamide monomers include: N-ethyl acrylamide, N-n-propyl (meth)acrylamide, i.e. N-n-propyl acrylamide and N-n-propyl methacrylamide, N-iso-propyl (meth)acrylamide, N-cyclopropyl (meth)acrylamide, N-N-diethyl acrylamide, N-methyl-N-ethyl acrylamide, N-methyl-N-n-propyl acrylamide, N-methyl-N-isopropyl acrylamide, N-acryloyl pyrrolidine, N-acryloyl piperidine, N-tetrahydrofurfuryl (meth)acrylamide, N-methoxypropyl (meth)acrylamide, N-ethoxypropyl (meth)acrylamide, N-isopropoxypropyl (meth)acrylamide, N-ethoxyethyl (meth)acrylamide, N-(2,2-dimethoxyethyl)-N-methyl acrylamide, N-methoxyethyl (meth)acrylamide, N-8-acryloyl-1,4-dioxa-8-aza-spiro[4,5]decane, N-(1,3-dioxolan-2-yl)-N-methyl acrylamide, N-1-methoxymethylpropyl (meth)acrylamide, N-1-methyl-2-methoxyethyl (meth)acrylamide, N-di(2-methoxyethyl)acrylamide, N-2-methoxyethyl-N-ethyl acrylamide, N-2-methoxyethyl-N-n-propyl acrylamide and N-methoxyethoxypropyl (meth)acrylamide. A homopolymer of these vinyl compounds has an intrinsic temperature of conversion into an insoluble form in water, referred to as the transition temperature in this invention. These vinyl compounds may be used either alone or as a combination of two or more thereof according to need.

In another embodiment of the invention, the thermoreversible thickener comprises a copolymer having thermomoreversibility of hydrophilicity and hydrophobicity obtained by the compolymerization of an acrylamide monomer which gives a homopolymer capable of exhibiting thermoreversibility of hydrophilicity and hydrophobicity in an aqueous solution with another vinyl compound. The acrylamide monomer is as defined above. As the other copolymerizable vinyl compound as the second component, hydrophilic vinyl compounds excepting the above mentioned thermoreversbile vinyl compounds, ionic vinyl compounds, or olephilic vinyl compounds can be used either alone or as a combination of two or more thereof according to need.

Typical examples of the hydrophilic vinyl compounds include N-methylol acrylamide, acrylamide, methacrylamide, N-methyl acrylamide, N,N-dimethyl acrylamide, acryloyl morpholine, diacetone acrylamide, hydroxyethyl (meth)acrylate, hydroxymethyl (meth)acrylate, hydroxypropyl (meth)acrylate, N-methoxypropyl (meth)-acrylamide, 2-methyl-5-vinyl pyridine and N-vinyl-2-pyrrolidone.

Typical examples of the ionic vinyl compounds include acrylic acid, methacrylic acid, 2-acrylamide-2 methylpropane sulfonic acid, styrene sulfonic acid, salts of these organic acids, amines such as N,N-dimethyl aminoethyl (meth)acrylate, N,N-diethylaminoethyl (meth)acrylate, N,N-dimethylaminopropyl (meth)-acrylamide and salts of these amines.

Typical examples of the oleophilic vinyl compounds include N-alkyl (meth)acrylamide compounds such as N-n-butyl (meth)acrylamide, N-sec-butyl (meth)acrylamide, N-tert-butyl (meth)acrylamide and N-n-hexyl (meth)acrylamide, (meth)acrylate compounds such as ethyl (meth)acrylate, methyl (meth)acrylate, butyl (meth)acrylate and glycidyl methacrylate, acrylonitirile, vinyl acetate, vinyl chloride, styrene and α-methylstryrene.

The copolymerization ratio of the vinyl compound as the second component is usually 90% by moles or less or, preferably, 60% by moles or less based on the total amount of the vinyl compounds depending on the particular combination thereof.

In a further different embodiment of the invention, the thermoreversible thickener comprises a copolymer having thermoreversibility of hydrophilicity and hydrophobicity obtained by the compolymerization of a vinyl compound which gives a homopolymer having no thermoreversibility of hydrophilicity and hydrophobicity in an aqueous solution with a vinyl compound which gives a water-insoluble homopolymer.

Typical examples of the vinyl compound which gives a homopolymer having no thermoreversibility of hydrophilicity and hydrophobicity in an aqueous solution include (meth)acrylamide, acrylamide derivatives such as N-methyl (meth)acrylamide, N,N-dimethyl (meth)acrylamide, N-methylol acrylamide and N,N-dimethylaminopropyl (meth)acrylamide, N-acryloyl morpholine, N-vinyl pyrrolidone and N,N-dimethylamino-ethyl (meth)acrylate.

Typical examples of the vinyl compound which gives a water-insoluble homopolymer include acrylamide derivatives such as N-n-butyl (meth)acrylamide, N-sec-butyl (meth)-acrylamide, N-tert-butyl (meth)acrylamide and N-n-hexyl (meth)acrylamide, diacetone acrylamide, N-tert-buytoxymethyl acrylamide, N-n-butoxypropyl (meth)acrylamide, N-2-ethylhexyloxypropyl acrylamide, N-furfuryl methacrylamide, N-acryloyl-2,6-dimethyl morpholine, N-methylthiopropyl acrylamide and N-chloropropyl acrylamide, (meth)-acrylate derivatives such as methyl (meth)acrylate, ethyl (meth)acrylate and butyl (meth)acrylate, acrylonitrile, vinyl acetate, vinyl chloride, styrene and α-methyl-styrene.

The copolymerization ratio of the vinyl compound which gives a homopolymer having no thermorever-sibility to the vinyl compound which gives a wa⁓er-insoluble homopolymer should be adequately selected depending on the combination of such vinyl compounds but is usually in the range of from 99:1 to 40:60 or, preferably, in the range of from 95:5 to 60:40. Each class of the vinyl compounds which give a homopolymer having no thermoreversiblity and the vinyl compounds which give a water-insoluble homopolymer respectively may be used either alone or as a combination of two or more thereof according to need.

Generally, the transition temperature of an aqueous solution of a copolymer having thermoreversiblity can be controlled by selecting the particular vinyl compounds as the constituents and the combination and compounding ratio thereof. In this case, many of the copolymers composed of two or more vinyl compounds, each of which gives a homopolymer having thermoreversibility, exhibit an additivity for the transition temperatures of the respective homopolymers multiplied by the respective weight fractions thereof. When copolymerization is performed with one or more vinyl compounds, each of which gives a homopolymer having thermoreversiblity, and one or more of the other copolymerizable vinyl compounds having no thermoreversibility, the transition temperature of the aqueous solution of the copolymer is modified by the introduction of such vinyl compounds. However, the introduction of a hydrophilic vinyl compound induces a tendency to increase the transition temperature and the introduction of a small amount of an oleophilic vinyl compound induces a tendency to decrease the transition temperature. A polymer having no thermoreversibility is obtained in each case by the introduction of a large amount of an oleophilic vinyl compound to render the compolymer insoluble in water and the introduction of a large amount of a hydrophilic vinyl compound to render the copolymer soluble in water.

Furthermore, as to the thickener of the present invention, the coverage of the temperature range from the initiation to the completion of transition is very narrow in an aqueous solution of the homopolymer. An aqueous dispersion admixed therewith has the characteristic that the solution exhibits a sudden change from a low-viscosity state to a highly viscous state within a narrow temperature range in the course of the thickening process by heating and, an aqueous solution of the copolymer has the characteristic that the solution has a broader temperature range than above and an aqueous dispersion admixed therewith also exhibits a gradual thickening through a broader temperature range than in the aqueous solution of a homopolymer in the course of temperature elevation for thickening. Accordingly, it is possible to appropriately control the thickening characteristic in accordance with the intended application.

Any conventional method of polymerization can be applied to the preparation of the homopolymer or copolymer having thermoreversibility of hydrophilicity and hydrophobicity as the thickener of the present invention. Applicable polymerization methods include the methods of solution polymerization, bulk polymerization, pearl polymerization and emulsion polymerization, the solution polymerization method usually being preferred. The solvent used in the solution polymerization is not limited specifically but is usually selected from water, alcohols such as methanol, ethanol and isopropanol, ketones such as acetone and methyl ethyl ketone, ethers such as tetrahydrofuran and dioxane, chlorinated hydrocarbons such as ethylene dichloride, chloroform and carbon tetrachloride, esters such as ethyl acetate and butyl acetate, aromatic hydrocarbons such as benzene and toluene, N,N-dimethyl formamide, dimethyl sulfoxide and acetonitrile and others. These solvents may be used either alone or as a combination of two or more thereof according to need. Of these solvents, aromatic hydrocarbons such as benzene and toluene, acetonitrile or water are particularly preferred.

The concentration of the monomers in the solution for the polymerization is usually from 0.1 to 90% by weight or, preferably, from 2 to 80% by weight based on the total weight of the polymerization mixture.

The polymerization reaction can be initiated by any conventional method including irradiation with high-energy radiation or electron beams, heating in the presence of a radical polymerization initiator and irradiation with light, e.g., ultraviolet light, in the presence of a photoinitiator. Typical examples of the radical polymerization initiator include azo compounds such as azobisisobutyronitrile, 2,2'-azobis-2,4-dimethyl valeronitrile, azobiscyclohexane carbonitrile, methyl azobisisobutyrate, azobisisobutyl amidine hydrochloride and azobiscyano valeric acid, persulfate compounds such as ammonium persulfate and sodium persulfate, peroxide compounds such as benzoyl peroxide, cumene hydroperoxide, tertbutyl hydroperoxide and peracetic acid, Redox-type initiator systems such as combinations of hydrogen peroxide and iron (II) salt, hydrogen peroxide and L-ascorbic acid, ammonium, persulfate and sodium sulfite, ceric sulfate and an alcohol, cerium ammonium nitrate, benzoyl peroxide and dimethylaniline. Among the above named initiators, azo compounds such as azobisisobutyronitrile and Redox-type initiators such as cerium ammonium nitrate are preferred.

The radical polymerization initiator is usually used in an amount of from 0.01 to 10% by weight or, preferably from 0.05 to 8% by weight based on the amount of the monomers. The polymerization temperature is selected depending on the polymerization method and the type of initiator but it is usually in the range of from 10 to 100°C or, preferably, from 20 to 80°C.

The copolymer with thermoreversiblity of hydrophilicity and hydrophobicity as the component of the thickener of the invention should have an intrinsic viscosity $[\eta]$ of at least 0.3 dl/g or, preferably, at least 0.5 dl/g at 27°C as measured in a solution of tetrahydrofuran, methanol or water. When the intrinsic viscosity of the copolymer is less than 0.3 dl/g under the above mentioned conditions, the thickening effect obtained thereby would not be satisfactory.

There is a gneeral relationship between the intrinsic viscosity and the thickening effect as shown below.

When the intrinsic viscosity is approximately in the range of from 0.3 to 1.0 dl/g, the thickening effect is not prominent, though dependent on the type of the aqueous dispersion, and such a thickener is satisfactorily used to impart a thickening effect of a not higher than medium degree to the aqueous dispersion. For example, thickeners in this condition are sometimes used as thickeners for coating compositions and adhesives or as rheology modifiers or water retention aids.

When the intrinsic viscosity exceeds about 1.0 dl/g, an outstandingly high thickening effect is obtained though dependent on the type of the aqueous dispersion, and such a thickener is satisfactorily used to impart a thickening effect of a high degree to the aqueous dispersions. For example, thickeners in this condition are used as gelation agents, immobilizing agents and thickening agents.

The thickener of the invention is usually used in an amount of from 0.001 to 30 parts by weight or, preferably, 0.01 to 20 parts by weight of the solid component to 100 parts by weight of the solid component in the aqueous dispersion. When the amount used exceeds 30 parts by weight, the water resistance of the objective article may decrease depending on the use of the aqueous dispersion so that coating compositions, adhesives and coated paper are accompanied by a difficulty in the practical use thereof. When the amount used is below 0.001 part by weight, on the other hand, the desired thickening effect cannot be obtained.

The thickener of the invention is admixed with the aqueous dispersion at a temperature not higher than the transition temperature thereof and the temperature of the dispersion admixed therewith is subsequently increased. However, the temperature for admixture should be selected taking into account the actual transition temperature of the thickener in the aqueous dispersion to which the thickener is added since the transition temperature may be influenced by various factors such as the kinds of salts, surface active agents, solvents and other constituents contained in the aqueous dispersion and the contents thereof.

When the thickener of the invention is added to an aqueous dispersion at a temperature higher than the transition temperature thereof, the thickening effect would sometimes not be satisfactorily exhibited. Excepting the requirement that the thickener of the invention is added to the aqueous dispersion at a temperature not higher than the transition temperature thereof followed by heating, the thickener of the invention can be used in a similar manner to that used for conventional thickeners. For example, the same thickening effect can be obtained by using a solution and by using a dried and pulverized product thereof as well.

The thickener of the invention may further contain or be admixed with antioxidants, ultraviolet absorbers, water-resistance improvers, preservatives, mildewcides, insecticidal and germicidal agents, dispersing agents, deodorants, perfumes, antifoam agents, extenders, dyes and pigments.

The thickener of the invention is applicable to water, an aqueous solution containing an inorganic or organic substance or an aqueous dispersion and found useful in modifying the viscosity characteristic to exhibit an effect widely differing in degree ranging from a slight increase in viscosity to a great increase in consistency to impart non-flowability. The above-mentioned aqueous dispersion includes naturally occurring and synthetic latices, various types of synthetic resin emulsions, aqueous solutions of inorganic salts, aqueous solutions of water-insoluble resins, aqueous slurries of inorganic and/or organic substances insoluble in water, colloidal dispersions or compositions as a mixture thereof. Examples of the aqueous dispersion for practical use include coating compositions, inks, tackifiers, adhesives, printing pastes, cosmetics, resin mortars, liquid mud-cement composites for boring and adhesives for carpet backing.

Furthermore, the thickener of the invention may be used effectively as a thickening agent for coating compositions for coated papers. Namely, coated papers are manufactured by applying a coating composition containing a pigment, binder and other additives to the surface of a base paper sheet followed by drying. It is essential in such a process to use a coating composition having a low viscosity and fluidity suitable for high-speed coating at the time of the coating works and early immobilization of the coating layer caused by suppressing migration of the pigment particles and binder in the coating layer in order to manufacture sheets of coated paper having excellent smoothness and printability. The transition temperature of the inventive thickener may possibly be lower than the drying temperature so that the coating composition is imparted with an advantageous characteristic of fluidity at the time of coating while immobilization of the coating layer is accelerated due to the thickening effect or gelation of the coating layer in the course of temperature elevation during the drying process after coating.

Typical examples of the aqueous emulsion as the object of practical application of the inventive thickener include acrylate-based or vinyl acetate-based emulsions such as Primal AC-61 (a registered trade name of an acrylate-based emulsion manufactured by Rohm & Haas Co.), Boncoat 6290 (a registered trade name of a vinyl acetate- and acrylate-based emulsion manufactured by Dai-nippon Ink & Chemicals, Inc.), Polysol AP-2666 (a registered trade name of an acrylate-based emulsion manufactured by Showa High Polymer Co.) and the like, coating compositions prepared by using these emulsions, SBR latices such as JSR 0696 or JSR 0692 (registered trade names of SBR-type latices manufactured by Japan Synthetic Rubber Co., Ltd.), Nippol LX407G (a trade name of an SBR-type latex manufactured by Nippon Zeon Co.,

Ltd.). L-1260 (a registered trade name of an SBR-type latex manufactured by Asahi Chemical Industries Co.) and the like, coating compositions for coated papers prepared by using these SBR latices, acrylate-based or ethylene-vinyl acetate copolymeric emulsions such as Sumika Flex (a registered trade name of an ethylene-vinyl acetate copolymeric emulsion manufactured by Sumitomo Chemical Co.), Bribine BPW-2200 (a registered trade name of an acrylate-based emulsion manufactured by Tokyo Ink Manufacturing Co.) and the like, adhesives and tackifiers prepared by using these emulsions, SBR-type latices such as JSR 0593 (a registered trade name of an SBR-type latex manufactured by Japan Synthetic Rubber Co.) and the like, adhesives for carpet backing prepared by using these latices and others.

The present invention is described in more detail in the following by way of reference examples for the preparation of the copolymers and application examples which should not be construed to limit the scope of the invention in any way.

Reference Example 1

A polymerization mixture was prepared by adding 7.50 g of N,N-dimethyl acrylamide, 2.50 g of N-tert-butyl acrylamide and 0.05 g of azobisisobutyronitrile to and dissolving them in 23.30 g of benzene contained in an ampule and the mixture was heated at 80 °C to effect polymerization for 8 hours after sealing of the ampule filled with nitrogen gas replacing air. After the end of the reaction time, benzene was evaporated from the polymerization mixture to dryness and the copolymer was purified by dissolving in acetone and pouring the acetone solution into n-hexane so as to precipitate the copolymer. The yield of the copolymer was 8.60 g. The viscosity of the copolymer was measured as a tetrahydrofuran solution at 27 °C using a dilution-type Ubbelohde viscosimeter to give an intrinsic viscosity of 4.10 dl/g. Further, the transition temperature of the copolymer 44 °C was determined by the measurement of the light transmission at a wavelength of 500 nm for a 1% by weight solution by increasing the temperature at a rate ot 1 °C/mtnute using a spectrophotometer equipped with an electronic temperature controller and a temperature programmer. The transition temperature ($T_L$) recorded was the temperaturea at which the light transmittance had decreased to a half of the initial value.

Reference Example 2

A polymerization mixture was prepared by adding 0.80 g of N-acryloyl morpholine, 0.20 g of diacetone acrylamide and 0.01 g of potassium persulfate to and dissolving them in 19.00 g of distilled water contained in an ampule and the mixture was heated at 75 °C to effect polymerization for 5 hours after sealing the ampule filled with nitrogen gas replacing air. After the end of the reaction time, water was evaporated from the polymerization mixture to dryness and the copolymer was purified by dissolving in acetone and pouring the acetone solution into n-hexane so as to precipitate the copolymer. The yield of the copolymer was 0.74 g. The viscosity of the copolymer was measured in the same manner as in Reference Example 1 to give an intrinsic viscosity of 1.21 dl/g. The transition temperature of the copolymer was 44 °C as determined in the same manner as in Reference Example 1.

Reference Example 3

A polymerization mixture was prepared by adding 9.00 g of N,N-dimethyl acrylamide, 1.00 g of styrene and 0.10 g of azobisisobutyronitrile to and dissolving them in 23.33 g of benzene contained in an ampule and the mixture was heated at 60 °C to effect polymerization for 8 hours after sealing the ampule filled with nitrogen gas replacing air. After the end of the reaction time, benzene was evaporated from the polymerization mixture to dryness and the copolymer was purified by dissolving in acetone and pouring the acetone solution into n-hexane so as to precipitate the copolymer. The yield of the copolymer was 8.35 g. The viscosity of the copolymer was measured in the same manner as in Reference Example 1 to give an intrinsic viscosity of 1.44 dl/g. The transition temperature of the copolymer was 51 °C as determined in the same manner as in Reference Example 1.

Reference Example 4

6

A polymerization mixture was prepared by adding 8.50 g of N,N-dimethyl acrylamide, 1.50 g of butyl acrylate and 0.10 g of azobisisobutyronitrile to and dissolving them in 23.33 g of benzene contained in an ampule and the mixture was heated at 80 ° C to effect polymerization for 8 hours after sealing the ampule filled with nitrogen gas replacing air. After the end of the reaction time, benzene was evaporated from the polymerization mixture to dryness and the copolymer was purified by dissolving in acetone and pouring the acetone solution into n-hexane so as to precipitate the copolymer. The yield of the copolymer was 6.55 g. The viscosity of the copolymer was measured in the same manner as in Reference Example 1 to give an intrinsic viscosity of 0.91 dl/g. The transition temperature of the copolymer was 41 ° C as determined in the same manner as in Reference Example 1.

Reference Example 5

A polymerization mixture was prepared in a conical flask of 100 ml capacity by mixing and dissolving 5.00 g of N-isopropyl acrylamide, 40 ml of benzene and 0.01 g of azobisisobutyronitrile and the polymerization reaction was effected at 25 ° C for 8 hours after replacement of air with nitrogen gas at room temperature. After the end of the reaction time, benzene was evaporated from the polymerization mixture to dryness and the polymer was purified by dissolving in acetone and pouring the acetone solution into n-hexane so as to precipitate the polymer. The yield of the copolymer was 4.9 g. The viscosity of the polymer was measured in the same manner as in Reference Example 1 to give an intrinsic viscosity of 2.05 dl/g. The transition temperature of the polymer was 80.4 ° C as determined in the same manner as in Reference Example 1.

Reference Example 6

A polymerization mixture was prepared in an ampule by mixing 4.72 g of N-ethyl-N-methyl acrylamide, 0.05 g of azobisisobutyronitrile and 20 ml of benzene and the ampule was sealed after deaeration and evacuation under freezing of the mixture therein with liquid nitrogen. The ampule was heated at 60 ° C for 90 minutes to effect the polymerization reaction. After the end of the reaction time, the polymer was purified by pouring the polymerization mixture into diethyl ether so as to precipitate the polymer. The yield of the thus obtained polymer was 8.50 g. The viscosity of the polymer was measured in the same manner as in Reference Example 1 to give an intrinsic viscosity of 1.64 dl/g. The transition temperature of the polymer was 56.0 ° C as determined in the same manner as in Reference Example 1.

Reference Example 7

A polymerization mixture was prepared in an ampule by mixing 7.52 g of N-methoxyethoxypropyl acrylamide, 0.03 g of azobisisobutyrontitrile and 20 ml of dioxane and the ampule was sealed after deaeration and evacuation under freezing of the mixture therein with liquid nitrogen. The ampule was heated at 60 ° C for 90 minutes to effect the polymerization reaction. After the end of the reaction time, the polymer was purified by pouring the polymerization mixture into diethyl ether so as to precipitate the polymer. The yield of the thus obtained polymer was 6.80 g. The viscosity of the polymer was measured in the same manner as in Reference Example 1 to give an intrinsic viscosity of 1.25 dl/g. The transition temperature of the polymer was 79 ° C as determined in the same manner as in Reference Example 1.

Reference Example 8

A polymerization mixture was prepared in an ampule by mixing 7.20 g of N-methoxyethoxypropyl acrylamide, 0.30 g of ethyl acrylate, 0.03 g of azobisisobutyronitrile and 20 ml of dioxane and the ampule was sealed after deaeration and evacuation under freezing of the mixture therein with liquid nitrogen. The ampule was heated at 60 ° C for 90 minutes to effect the polymerization reaction. After the end of the reaction time, the copolymer was purified by pouring the polymerization mixture into diethyl ether so as to precipitate the copolymer. The yield of the thus obtained copolymer was 5.70 g. The viscosity of the polymer was measured in the same manner as in Reference Example 1 to give an intrinsic viscosity of 1.00 dl/g. The transition temperature of the copolymer was 77 ° C as determined in the same manner as in Reference Example 1.

Reference Example 9

A polymerizatidn mixture was prepared in a conical flask of 100 ml capacity by mixing and dissolving 3.24 g of N-methyl acrylamide, 1.76 g of N-acryloyl piperidine, 40 ml of dioxane and 0.02 g of azobisisobutyronitrile and the polymerization reaction was effected at 60 °C for 8 hours after replacement of air in the flask with nitrogen gas at room temperature. After the end of the reaction time, dioxane was evaporated from the polymerization mixture to dryness and the copolymer was purified by dissolving in acetone and pouring the acetone solution into n-hexane so as to precipitate the copolymer. The yield of the copolymer was 3.50 g. The viscosity of the copolymer was measured in the same manner as in Reference Example 1 to give an intrinsic viscosity of 0.91 dl/g. The transition temperature of the polymer was 56 °C as determined in the same manner as in Reference Example 1.

Reference Example 10

A polymerization mixture was prepared in a conical flask of 100 ml capacity by mixing and dissolving 0.20 g of N,N-dimethyl acrylamide, 2.00 g of N-methyl-N-ethyl acrylamide, 40 ml of methanol and 0.01 g of azobisisobutyronitrile and the polymerization reaction was effected at 60 °C for 8 hours after replacement of air in the flask with nitrogen gas at room temperature. After the end of the reaction time, methanol was evaporated from the polymerization mixture to dryness and the copolymer was purified by dissolving in acetone and pouring the acetone solution into n-hexane so as to precipitate the copolymer. The yield of the copolymer was 2.10 g. The viscosity of the polymer was measured in the same manner as in Reference Example 1 to give an intrinsic viscosity of 0.68 dl/g. The transition temperature of the copolymer was 60 °C as determined in the same manner as in Reference Example 1.

Reference Example 11

A polymerization mixture was prepared in a conical flask of 100 ml capacity by mixing and dissolving 1.79 g of N-isopropyl acrylamide, 0.28 g of hydroxyethyl acrylate, 40 ml of methanol and 0.01 g of 2,2'-azobis-2,4-dimethylvaleronitrile and the polymerization reaction was effected at 60 °C for 8 hours after replacement of air in the flask with nitrogen gas at room temperature. After the end of the reaction time, methanol was evaporated from the polymerization mixture to dryness and the copolymer was purified by dissolving in acetone and pouring the acetone solution into n-hexane so as to precipitate the copolymer. The yield of the copolymer was 1.5 g. The viscosity of the polymer was measured in the same manner as in Reference Example 1 to give an intrinsic viscosity of 0.51 dl/g. The transition temperature of the polymer was 40 °C as determined in the same manner as in Refernece Example 1.

Example 1

An SBR latex (JSR 0696, a trade name by Japan Synthetic Rubber Co.) was admixed with 0.5% of the polymer obtained in Reference Exmaple 1 on a solid basis at 25 °C to give a uniform mixture by dissolving. Thereafter, viscosity of the latex was measured while it was heated at a rate of temperature elevation of 1 °C per minute, starting from 25 °C, using a Brookfield-type viscosimeter with a rotor rotating at 60 rpm. The viscosity measurement was performed 1 miute after the respective temperature had been reached. Separately, the same measurement as above was repeated except that the viscosity measurement was performed after keeping the mixture for 30 minutes at the respective temperature. The results of the measurements are shown in Table 1.

As is understood from the results in Table 1, the thickener of the invention exhibits a high sensitivity to thickening response.

Table 1

| Temperature, °C | Viscosity after 1 minute, cps | Viscosity after 30 minutes, cps |
|---|---|---|
| 25 | 750 | 740 |
| 30 | 830 | 830 |
| 40 | 900 | 920 |
| 50 | 1100 | 1150 |
| 60 | 1250 | 1280 |
| 70 | 1300 | 1320 |

Examples 2 and 3 and Comparative Examples 1 and 2

An acrylate-type semi-glossy paint composition was prepared each by uniformly blending 81.7 g of water, 25.0 g of propylene glycol, 8. 0 g of Nopco Sperse 44-C, 2.0 g 5 of Foamaster VL (anti-foaming agent manufactured by Sun Nopco Ltd.), 2.0 g of Highonic PE-40, 22.5 g of Nopcoside N-40D, 225.0 g of titanium dioxide, 200.0 g of clay, 30.0 g of Celite, 325.6 g of acrylic emulsion (Primal AC-61), 12.0 g of Texanol, 2.0 g of Foamaster VL, 3.9 g of Butyl Carbitol and 225.0 g of water. The paint composition was admixed with one or a combination of the thickeners including the polymer obtained in Reference Example 3, the polymer obtained in Reference Example 6, a polyether-based thickening agent (SN-Thickner 612, a Product by Sun Nopco Ltd.) and a hydroxyethyl cellulose in amounts indicated in Table 2 below.

The thus obtained compositions were subjected to the measurement of the viscosity at temperatures of 25 ° C, 35 ° C and 60 ° C to give the results shown in Table 2.

The Paint compositions were applied to a slate board to evaluate the leveling Property and the color acceptance and applied to a glass plate to measure the 60° gloss, i.e. 60-degree light reflectance.

Experimental results are shown in Table 2.

T a b l e  2

| | | Example | | Comparative Example | |
|---|---|---|---|---|---|
| | | 2 | 3 | 1 | 2 |
| Thickner, g | Reference Example 2 | 2.4 | - | - | - |
| | Reference Example 6 | - | 2.4 | - | - |
| | Polyether-based | 15.5 | 15.5 | 15.5 | - |
| | Hydroxy-ethyl cellulose | - | - | - | 3.5 |
| Viscosity of paint, KU | 25 °C | 103 | 101 | 99 | 100 |
| | 35 °C | 104 | 99 | 94 | 98 |
| | 60 °C | 106 | 105 | 90 | 95 |
| Leveling property *1 | | A | A | A | B |
| Color acceptance, property *2 | Touch-up | 0.41 | 0.38 | 0.55 | 0.35 |
| | Soptting | 0.35 | 0.31 | 0.50 | 0.24 |
| | Rubbing | 0.44 | 0.51 | 1.21 | 0.38 |
| 60° Gloss *3 | | 67 | 68 | 69 | 60 |

*1    The viscosity of thickened paint was adjusted to 70 KU  by adding water and the paint was applied to a slate board by brushing to visually examine the brush marks.

    A : little

    B : slight

    C : strong

*2    The color differences of the coated surfaces after being subjected to touch-up, spotting or rubbing in comparison with the one-coat finished surface were evaluated in NBS Units using a color difference meter (a digital color measuring-difference meter manufactured by Tokyo Denshoku Co.)

*3    The paint was applied to a glass plate using an applicator to give a film thickness of 0.152 mm followed by drying for 24 hours at room temperature and the value of 60-degree gloss was measured using a gloss meter manufactured by Tokyo Denshoku Co.

Examples 4 and 5 and Comparative Examples 3 and 4

Coating materials for coated paper were prepared each by uniformly blending 49.1 g of water, 0.5 g of a dispersing agent (SN-Dispersant 5040, manufactured by Sun Nopco Ltd.), 0.1 g of sodium hydroxide, 0.1 g of Nopco DF122 (an anti-foaming agent manufactured by Sun Nopco Ltd.), 70.0 g of clay, 15.0 g of precipitated calcium carbonate, 15.0 g of heavy calcium carbonate, 16.7 g of a 30% aqueous dispersion of oxidized starch, 27.0 g of an SBR latex (solid content 48%) and 1.0 g of Nopcoat C-104 (a lubricant manufactured by Sun Nopco Ltd.) along with addition of 0.3 g of the polymer obtained in Reference Example 1 (Example 4), the polymer obtained in Reference Example 7 Example 5) or a carboxymethyl cellulose (Comparative Example 3) as a thickener or without addition of any thickner (Comparative Example 4).

The thus obtained coating compositions were each applied to a sheet of paper according to the condition shown in Table 3 and the physical properties of the coated paper sheets were measured after drying. The results of testing are shown in Table 4.

Table 3

| Base paper | form paper |
|---|---|
| Application | Helicoater, 800 meters/minute |
| Drying | at 150 °C for 30 seconds |
| Coating amount | 14 g/m² as dried |
| Calendering | Super Calender, 90 kg/cm, 55 °C, 2 passes |

Table 4

| | | | Example | | Comparative Example | |
|---|---|---|---|---|---|---|
| | | | 4 | 5 | 3 | 4 |
| Property of coating material | Brook-field viscosity, cps, at | 25 °C | 3000 | 1200 | 1600 | 1050 |
| | | 60 °C | | >10000 | 1200 | 610 |
| | | 80 °C | 7000 | | 1000 | 520 |
| | Water retention, seconds | | 30 | 31 | 33 | 21 |
| Property of coated paper | Air permeability, seconds | | 970 | 970 | 960 | 970 |
| | Smoothness, seconds | | 1400 | 1500 | 1300 | 1350 |
| | Brighteness, % | | 83.1 | 83.1 | 83.2 | 83.0 |
| | K&N ink receptivity, % | | 18.2 | 18.5 | 18.1 | 17.5 |
| | Sheet gloss, % | | 82.5 | 84.8 | 81.2 | 81.0 |
| | Printed gloss, % | | 85.6 | 86.6 | 83.2 | 82.9 |
| | Ink receptivity in wet, 5-rating method | | 4 | 4 | 3.5 | 3.5 |

Note

1) Water retention :

    static ring-cell type water-retention tester

    (S.D. Warren's method)

2) Printing test : Type RI-2 (Akira Manufacturing Co.)

3) Brightness and gloss :

    digital colorimetric color-difference meter

    (Tokyo Denshoku, Model TC-500)

4) K&N ink receptivity :

    Indicated by the lowering ratio of the brightness

    measured after ink-reception in comparison with

    the brightness before testing

5) Air permeability and smoothness :

    Ouken Model Air Permeability-Smoothness Tester

    (Asahi Seiko Co.)

6) Ink receptivity in wet : evaluation by 5-rating method

    5 ... good; 1 ... poor

Example 6

An acrylate-type emulsion (Primal AC-61, manufactured by Rohm & Haas Co.) was admixed with the polymer obtained in Reference Example 5 in an amount of 0.1% by weight on a solid basis at 18 ° C to give a uniform mixture by dissolving. After mixing, the temperature of the emulsion was elevated at a rate of 1 ° C per minute and viscosity measurement was performed in the same manner as in Example 1 to give the results shown in Table 5.

As in understood from the results in Table 5, the thickener of the invention exhibits a high sensitivity to thickening response. Also, it can be understood that the transition temperature of the polymer in Reference Example 5, which has inherently a transition temperature of 30.4 ° C, used herein is changed to 18 to 19 ° C in an emulsion which is a mixture containing a surface active agent and other ingredients.

13

Table 5

| Temperature, °C | Viscosity after 1 minute, cps | Viscosity after 30 minutes, cps |
|---|---|---|
| 13 | 52 | 51 |
| 14 | 50 | 50 |
| 15 | 50 | 51 |
| 16 | 58 | 59 |
| 17 | 200 | 200 |
| 18 | 4,000 | 3,980 |
| 19 | >30,000 (non-flowable) | >30,000 (non-flowable) |
| 20 | >30,000 (non-flowable) | >30,000 (non-flowable) |
| 30 | >30,000 (non-flowable) | >30,000 (non-flowable) |
| 40 | >30,000 (non-flowable) | >30,000 (non-flowable) |
| 50 | >30,000 (non-flowable) | >30,000 (non-flowable) |

Then, the temperature of this thickened emulsion was lowered at a rate of 1 °C Per minute from 50 °C to 18 °C and measurements were carried out for the viscosity thereof. The viscosity was measured 1 minute after the moment when the respective temperature had been reached. Separately, the same thickened emulsion was kept at 50 °C for 80 minutes and then cooled down to room temperature to measure the viscosity thereof 30 minutes after the moment when the respective temperature had been reached. The results are shown in Table 6.

Table 6

| Temperature, °C | Viscosity after 1 minute, cps | Viscosity after 30 minutes, cps |
|---|---|---|
| 50 | >30,000 (non-flowable) | >30,000 (non-flowable) |
| 40 | >30,000 (non-flowable) | >30,000 (non-flowable) |
| 30 | >30,000 (non-flowable) | >30,000 (non-flowable) |
| 20 | >30,000 (non-flowable) | >30,000 (non-flowable) |
| 19 | >30,000 (non-flowable) | >30,000 (non-flowable) |
| 18 | 6,000 | 4,100 |
| 17 | 220 | 210 |
| 16 | 65 | 57 |
| 15 | 55 | 50 |
| 14 | 50 | 50 |
| 13 | 52 | 51 |

Example 7

An acrylate-type emulsion (Primal AC-61, supra) was admixed with the polymer obtained in Reference Example 9 in an amount of 0.1% by weight on a solid basis at 30 ° C to give a uniform mixture by dissolving. After mixing, viscosity measurement was carried out for this emulsion at several temperatures using a Brookfield-type viscosimeter with a rotor rotating at 60 rpm. The results of measurements are shown in Table 7.

Table 7

| Temperatutre, °C | Viscosity of emulsion, cps |
|---|---|
| 30 | 50 |
| 40 | 55 |
| 50 | 72 |
| 53 | 120 |
| 60 | 770 |
| 70 | 1600 |
| 75 | 2200 |

As is explained fully in detail in the above, the thermoreversible thickener of the invention comprising a homopolymer or copolymer having thermoreversibility of hydrophilicity and hydrophobicity has an effect of enabling controlling of the viscosity in a wide range from the low viscosity to a highly viscous condition or, furthermore, to a non-fluid c<ndition in a broad temperature range when it is added to various types of aqueous dispersions. The thickener of the invention has a characteristic of easy setting of the temperature for beginning of thickening, high sensitivity to the thickening response in the thickening process of·aqueous dispersions, absence of lowering of the viscosity of thickened aqueous dispersions by temperature elevation and so on. Accordingly, the inventive thickner provides an improvement in respect of the disadvantages inherent in conventional thickeners or gelation agents.

In the application of the thickener of the invention to a coating material for coated paper, the coating material can be imparted with excellent water retention and fluidity without thickening by being mixed with the thickener at a temperature not exceeding the transition temperature thereof.

Therefore, it is evident that such a coating material has excellent applicability to paper coating. Coated paper having excellent smoothness and bulkiness can be obtained due to quick immobilization of the coating layer by drying at a temperature exceeding the transition temperature. In addition, effects of increasing the sheet gloss, printed gloss and ink receptivity are obtained.

Additionally, in the case of applying the thickener of the invention to coating materials for general use, the coating materials are imparted with a desired degree of thickening effect and, at the same time, an excellent rheological behavior including leveling, fluidity and the like, color acceptance, stability of dispersion, water retention and so on without decreasing the gloss of the coating film.

The above described advantageous improvement in the performance of an aqueous dispersion can never be obtained by using any conventional thickeners.

**Claims**

1. A thermoreversible thickener for an aqueous dispersion which thickener comprises a polymer having thermoreversibility of hydrophilicity and hydrophobicity which is the polymerization product of at least one acrylamide monomer capable of giving a polymer exhibiting thermoreversibility of hydrophilicity and hydrophobicity in an aqueous medium.

2. A thermoreversible thickener for an aqueous dispersion which thickener comprises a copolymer having thermoreversibility of hydrophilicity and hydrophobicity obtained by the copolymerization of an acrylamide monomer capable of giving a homopolymer which exhibits thermoreversibility of hydrophilicity and hydrophobicity in an aqueous solution and another vinyl compound incapable of giving a homopolymer which exhibits thermoreversibility of hydrophilicity and hydrophobicity.

15

3. A thermoreversible thickener for an aqueous dispersion which thickener comprises a copolymer having thermoreversibility of hydrophilicity and hydrophobicity obtained by the copolymerization of a first vinyl compound capable of giving a homopolymer which does not exhibit thermoreversibility of hydrophilicity and hydrophobicity in an aqueous medium and a second vinyl compound which gives a homopolymer insoluble in water.

4. A thermoreversible thickener as claimed in any one of the preceding claims in which the polymer having thermoreversibility of hydrophilicity and hydrophobicity in an aqueous solution has an intrinsic viscosity of at least 0.3 dl/g.

5. A coating composition for coated paper which comprises, in admixture in an aqueous solution:

(a) a pigment;

(b) a water-soluble polymer as a binder of the pigment; and

(c) a thermoreversible thickener for an aqueous dispersion as claimed in any one of the preceding claims.